**Europäisches Patentamt**

**European Patent Office**

**Office européen des brevets**

(19)

(11) Veröffentlichungsnummer: **0 247 362**
**A2**

# (12) EUROPÄISCHE PATENTANMELDUNG

(21) Anmeldenummer: **87105999.4**

(22) Anmeldetag: **24.04.87**

(51) Int. Cl.⁴: **A 61 K 47/00**, A 61 L 15/03

(30) Priorität: **25.04.86  DE 3614095**

(43) Veröffentlichungstag der Anmeldung: **02.12.87**
**Patentblatt 87/49**

(84) Benannte Vertragsstaaten: **AT BE CH DE ES FR GB GR IT LI LU NL SE**

(71) Anmelder: **GÖDECKE AKTIENGESELLSCHAFT, Salzufer 16, D-1000 Berlin 10 (DE)**

(72) Erfinder: **Ambrosch, Walter, Karl-Schmidt-Strasse 2a, D-7830 Emmendingen 15 (DE)**
Erfinder: **Schneider, Michael, Dr., Brestenbergstrasse 6, D-7809 Denzlingen (DE)**

(54) **Oxyalkylcellulose enthaltende Gelzubereitung.**

(57) Die Erfindung betrifft eine pharmazeutische wäßrige Gelzubereitung für die topische Anwendung, welche als Trägerstoff für die pharmazeutischen Wirkstoffe niedere Oxyalkylcellulose enthält. Es hat sich gezeigt, daß solche Trägerstoffe keine die Wundheilung verhindernden Gewebereizungen hervorrufen.

EP 0 247 362 A2

ACTORUM AG

0247362

BESCHREIBUNG

Für die Entwicklung von topisch anwendbaren, die Wundheilung
fördernden Gelzubereitungen ist es wichtig ein Trägermaterial bereitzustellen, das auf den Verlauf der Wundheilung einen positiven Effekt
ausübt, zumindest aber physiologisch keine Reizwirkung oder eine
den Heilungsprozess verzögernde Wirkung hat.

Dies ist nicht einfach, da bei offenen Wunden das Gewebe äußerst
empfindlich ist und auch bei von sich nicht aggressiven Fremdstoffen
gereizt wird, was den Heilungsprozess verzögert und beim Patienten
Schmerzen verursacht.

Aufgabe der Erfindung ist es, einen Trägerstoff für topische Gelzubereitungen bereitzustellen, der eine solche Reizwirkung auf verletztes
Gewebe von warmblutigen Tieren und Menschen nicht ausübt und damit
die Wundheilung fördert.

Es wurde gefunden, daß Oxyalkylcellulose einen solchen Trägerstoff
darstellt, der in Form einer wäßrigen Gelzubereitung (Hydrogel) keine
Reizwirkung auf das Gewebe ausübt und unter Umständen sogar selbst
ohne Wirkstoffe, einen heilungsfördernden Effekt zeigt.

Zweckmäßig wird dem erfindungsgemäßen Hydrogel jedoch ein heilungsfördernder Wirkstoff zugegeben, der wiederum keine Reizwirkung auf
das Gewebe haben sollte, um die Vorteile des Trägermaterials nicht
zunichte zu machen.

Beispielsweise eignen sich hierfür hervorragend Wirkstoffgemische nach
DE-PS 34 16 777, da diese in isotoner Zusammensetzung sowohl eine die
Wundheilung stark fördernde Wirkung besitzen und gleichzeitig vom
Gewebe ohne jede Reizung vertragen werden.

0247362

Als Oxyalkylcellulose werden solche Cellulosen verstanden die niedere Alkylreste, d.h. solchen, die jeweils 1 bis 4, bevorzugt 1 bis 2 Kohlenstoffatome bzw. Wasserstoff und einen niederen Alkylrest aufweisen.

Hierzu gehören z.B.
Methylhydroxymethylcellulose
Methylhydroxypropylcellulose
Butylhydroxymethylcellulose
Ethylhydroxypropylcellulose
Hydroxymethylcellulose
Hydroxyethylcellulose
Hydroxypropylcellulose
Hydroxybutylcellulose
und bevorzugt Methylhydroxyethylcellulose.

Unter Cellulosen werden solche mit einer Polymerisationszahl von 120 bis 1200 verstanden, wie sie z.B. unter dem Handelsnamen "Tylose" ® vertrieben werden.

Diese bilden mit Wasser klare Gele, die streichfähig sind und in steriler und möglichst isotoner Form ohne weiteres zusammen mit entsprechend dosierten Wirkstoffen auf Wundoberflächen aufgebracht werden können.

Die Gele können zubereitet werden, indem man die Oxyalkylcellulose langsam und unter Rühren in warmes Wasser einträgt und bis zur Homogenität quellen läßt. Dem Wasser können vorher gegebenenfalls Mittel zur Einstellung der Isotonie, z.B. Kochsalz, Glycerin oder Sorbit, sowie Konservierungsmittel, wie z.B. PHB-Ester zugegeben werden.

Gegenstand der Erfindung ist somit eine pharmazeutische wäßrige Gelzubereitung zur topischen Anwendung, die dadurch gekennzeichnet ist, daß sie als Trägerstoff für die pharmazeutischen Wirkstoffe eine $C_1$ bis $C_6$-Alkylhydroxy-$C_1$ bis $C_6$-alkyl-cellulose oder eine Hydroxy-$C_1$ bis $C_6$-alkyl-cellulose enthält.

Die Gelzubereitung kann selbstverständlich je nach Anwendungsziel auch mit anderen Zubereitungen, wie z.B. Öl in Wasser - oder Wasser in Öl-Emulsionen gemischt werden, um beispielsweise der Zubereitung einen gewissen Grad von Lipophilität zu geben, die etwa das Verbacken oder Verkrusten der Wundoberfläche verhindert.

0247362

Man wird auch darauf zu achten haben, daß bei der Zubereitung ein für die Wundheilung günstiger pH-Bereich von etwa 3,5 bis 7,5, bevorzugt 4-7 nicht unter- bzw. überschritten wird. Bei ersten Versuchen hat sich ein sorbithaltiges Gel mit pH 4 als sehr gut verträglich erwiesen.

Die Viskosität sollte anhand des Verhältnisses Cellulose/Wasser so eingestellt werden, daß das Gel auf der warmen Hautoberfläche nicht zu leicht zerfließt und sich andererseits leicht zerteilen läßt. Eine Viskosität von 30 bis 60 Pa.s scheint hier günstig zu sein.

Beispiel 1

Herstellung des wirkstofffreien Gels

75 g 70 Gew%ige Sorbitlösung werden mit 2 g PHB-Ester als Konservierungsmittel in 883 ml Wasser gelöst. Diese Lösung wird auf 60°C erwärmt und unter Rühren langsam mit 40 g Methylhydroxyethylcellulose versetzt. Man rührt 1 bis 2 Stunden bis zur Homogenität und kühlt über diesen Zeitraum auf Raumtemperaturen.

Man erhält ein transparentes Gel vom pH 4 und einer Viskosität von 50 Pa.s. (bei 20°C) folgender Zusammensetzung:

| | |
|---|---|
| Methylhydroxyethylcellulose | 4,00 % |
| Sorbitlösung 70 % | 7,50 % |
| Konservierungsmittel | 0,20 % |
| Gereinigtes Wasser | ad 100,00 % |

B e i s p i e l   2

Pharmazeutische Zubereitung mit einem $K^+/Ca^{++}$-Gemisch als Wirkstoff
(nach DE-PS 34 16 777)

Gemäß Beispiel 1 wird eine 60$^o$C warme wäßrige Lösung mit 30 mmol $CaCl_2$, und 40 mmol KCl als Wirkstoffgemisch sowie mit Sorbit zur Einstellung der Isotonie und PHB-Ester als Konservierungsmittel hergestellt und mit Methylhydroxyethylcellulose versetzt. Man erhält ein Gel mit einem pH-Wert von 4,0 und einer Viskosität von 40 Pa.s.

Das Gel hat folgende Zusammensetzung:

| | | |
|---|---|---|
| Calciumchlorid | 30 mmol... | 0,44 % |
| Kaliumchlorid | 40 mmol... | 0,30 % |
| Methylhydroxyethylcellulose | | 4,00 % |
| Sorbitlösung 70 % | | 2,80 % |
| Konservierungsmittel | | 0,20 % |
| Gereinigtes Wasser | | ad 100,00 % |

Beispiel 3

Pharmazeutische Zubereitung mit einem $K^+$/$Ca^{++}$-Gemisch als Wirkstoff (nach DE-PS 34 16 777)

Gemäß Beispiel 1 wird eine 60°C warme wäßrige Lösung mit 30 mmol $CaCl_2$, und 40 mmol KCl als Wirkstoffgemisch sowie mit Sorbit zur Einstellung der Isotonie und PHB-Ester als Konservierungsmittel hergestellt, mit Methyl-hydroxyethylcellulose versetzt, die Salzsäure zugesetzt und unter Rühren abgekühlt. Man erhält ein Gel mit einem pH-Wert von 4,0 und einer Viskosität von 35 Pa.s.

Das Gel hat folgende Zusammensetzung:

| | | |
|---|---|---|
| Calciumchlorid | 30 mmol... | 0,44 % |
| Kaliumchlorid | 40 mmol... | 0,30 % |
| Methylhydroxyethylcellulose (Tylopur®) | | 4,00 % |
| Salzsäure 0,01 N | | 4,00 % |
| Sorbitlösung 70 % | | 2,80 % |
| Konservierungsmittel | | 0,20 % |
| Gereinigtes Wasser | | ad 100,00 % |

PATENTANSPRÜCHE

1.) Pharmazeutische wässrige Gelzubereitung zur topischen Anwendung, dadurch gekennzeichnet, daß sie als Trägerstoff für die pharmazeutischen Wirkstoffe eine $C_1$ bis $C_6$-Alkyl-hydroxy-$C_1$-$C_6$-alkyl-cellulose oder eine Hydroxy-$C_1$ bis $C_6$-alkyl-cellulose enthält.

2.) Zubereitung nach Anspruch 1, dadurch gekennzeichnet, daß diese als Trägerstoff Methylhydroxyethylcellulose enthält.

3.) Verfahren zur Verwendung von $C_1$ bis $C_6$-Alkyl-hydroxy-$C_1$-$C_6$-alkyl-cellulose und/oder Hydroxy $C_1$ bis $C_6$-alkylcellulose als Trägerstoff für die Herstellung pharmazeutischer wässriger Gelzubereitungen zur topischen Anwendung.

4.) Verfahren nach Anspruch 3 zur Verwendung von Methylhydroxyethyl-cellulose.